Europäisches Patentamt

European Patent Office

Office européen des brevets

(11) Publication number: **0 252 666 B1**

# EUROPEAN PATENT SPECIFICATION

(45) Date of publication of patent specification: **23.06.93**  (51) Int. Cl.⁵: **C12N  15/56**, C12P 19/14

(21) Application number: **87305781.4**

(22) Date of filing: **30.06.87**

(54) **Chimeric Enzymes.**

(30) Priority: **30.06.86 DK 3111/86**

(43) Date of publication of application:
**13.01.88 Bulletin  88/02**

(45) Publication of the grant of the patent:
**23.06.93 Bulletin  93/25**

(84) Designated Contracting States:
**AT BE CH DE ES FR GB GR IT LI LU NL SE**

(56) References cited:
**EP-A- 0 180 952**
**EP-A- 0 208 491**
**WO-A-85/00382**

**JOURNAL OF BIOCHEMISTRY Volume 98,
no.5, November 1985,Tokyo Japan,pages
1147-1156; T.Yuuki et al**

**JOURNAL OF BIOCHEMISTRY Volume 98,
no.1, July 1985,Tokyo Japan,pages 95-103;
H.Ihara**

(73) Proprietor: **NOVO NORDISK A/S
Novo Allé
DK-2880 Bagsvaerd(DK)**

(72) Inventor: **Diderichsen, Borge Krag
Estersvej 32
DK-2900 Hellerup(DK)**
Inventor: **Outtrup, Helle
Syvendehusvej 46
DK-2750 Ballerup(DK)**
Inventor: **Schülein, Martin
Wiedeweltsgade 51
DK-2100 Kobenhavn 0(DK)**
Inventor: **Norman, Barrie Edmund
Kielshoj 118
DK-3520 Farum(DK)**

(74) Representative: **Brown, John David et al
FORRESTER & BOEHMERT Franz-
Joseph-Strasse 38
W-8000 München 40 (DE)**

**Description**

This invention relates to starch hydrolysing enzymes. More specifically, the present invention is directed to chimeric alpha-amylases, to processes for preparing such chimeric alpha-amylases and to the use thereof for the overall enzymatic conversion of starch into high DX syrups, the term DX meaning percentage by weight of dextrose (D-glucose) calculated on the basis of dry substance (DS) of the syrup.

2. BACKGROUND OF THE INVENTION

The overall enzymatic process generally adopted by manufacturers of high DX syrups from starch entails two-stages: liquefaction and saccharifaction. The first step, the liquefaction, involves the hydrolysis of starch into a mixture of oligosaccharides, the so called maltodextrins. This process is catalyzed by alpha-amylases at a temperature of at least 75°C, preferably at about 90°C or by a jet-cooking process wherein the starch slurry is heated for at least several minutes to 105-110°C, usually with a single dose of alpha-amylase, and then held at about 90°C for at least one hour.

A variety of microbial, particularly bacterial, alpha-amylases are commercially available for the liquefaction process, for example BAN™ (from Bacillus amyloliquefaciens and TERMAMYL® (from Bacillus - licheniformis), both supplied by NOVO INDUSTRI A/S, Denmark, and THERMOLASE™ (from Bacillus stearothermophilus) available from Enzyme Development Corporation, N.Y., U.S.A. While BAN alpha-amylase is only stable up to about 85°C and hence barely suitable for the jet-cooking process, both the TERMAMYL and THERMOLASE enzymes are well adapted for this almost globally preferred mode of starch liquefaction because they are heat stable.

The saccharification step, in which the maltodextrins are converted into dextrose, is mostly catalyzed by a glucoamylase enzyme. Commercial glucoamylase preparations, usually derived from Aspergillus or Rhizopus species, are available from various manufacturers, e.g. as AMG™ 200L, a product obtained from Aspergillus niger and manufactured by NOVO INDUSTRI A/S, Denmark.

With a view to further increasing the dextrose yield from 30 - 40 percent by weight DS maltodextrin solutions it has become customary to conduct the saccharification process with glucoamylase in the presence of a debranching enzyme in order to facilitate the hydrolysis of branched oligosaccharides originating from the amylopectin portion of starch. One such debranching enzyme with maximum activity in the same pH and temperature ranges as glucoamylase is disclosed in European Patent Application No. 82302001.1 (Publication No. 0063909). The debranching enzyme is marketed by NOVO INDUSTRI A/S, Denmark, either as such under the proprietary name, PROMOZYME, or as a composition with suitable admixture of glucoamylase under the proprietary name DEXTROZYME.

Unfortunately, the otherwise very favorable combination of B. licheniformis alpha-amylase for liquefaction and glucoamylase-PROMOZYME for saccharification in the conversion of starch to high DX syrups entails an inconvenience. It has been observed that the presence of residual alpha-amylase activity from the liquefaction stage has a negative effect on the maximum DX obtainable by saccharification with glycoamylase-PROMOZYME. The problem is greatest with the thermostable B. licheniformis alphaamylase which is still active at the preferred conditions for saccharification (of about pH 4.6 and temperature of about 60°C, respectively). A remedy has been devised consisting of inactivation of the alpha-amylase prior to saccharification by acidification of the liquefied starch to a pH below 4.5 while maintaining a temperature of at least 90°C. Following inactivation of the alpha-amylase, the temperature and pH are adjusted to saccharification conditions, meaning that the pH has to be brought up to about 4.5. This additional pH adjustment inevitably increases the salt content of the syrup and hence the expenses connected with de-salting the final syrup.

The object of the present invention is to overcome the above-mentioned inconveniences still associated with the use of B. licheniformis alpha-amylase for the conversion of starch into a high DX syrup. This and other objects which will be dealt with subsequently in this specification are attained by conducting the liquefaction process with a novel type of alpha-amylase.

3. SUMMARY OF THE INVENTION

The chimeric alpha-amylase enzymes of the invention comprise all or portions of the amino terminus of the alpha-amylase derived from B. amyloliquefaciens joined to the carboxy terminus of the alpha-amylase derived from B. licheniformis. Briefly stated, the present invention provides chimeric alpha-amylases, which are thermostable and exhibit a reduced negative effect on the use of A. niger glucoamylase and B. acidopullulyticus pullulanase for the saccharification of starch, having the general formula I

2

**(I)**

**Q-R-L**

in which Q is a N-terminal part of from 55 to 60 amino acid residues which is at least 75 percent, preferably at least 80 percent, and more preferably at least 90 percent homologous to the 57 N-terminal amino acid residues in the Bacillus amyloliquefaciens alpha-amylase (Takkinen, et al., 1983, J.Biol.Chem. 258:1007-1013);

R is a part of the general formula Ia:

**(Ia)**

58      60                  65                70

$$\text{Pro-Tyr-Asp-Leu-Tyr-Asp-Leu-Gly-Glu-Phe-}X_8\text{-Gln-Lys-}$$

75                    80

$$\text{Gly-Thr-Val-Arg-Thr-Lys-Tyr-Gly-Thr-Lys-}X_9\text{-Glu-Leu-}$$

85           88

$$\text{Gln-}X_{10}\text{-Ala-Ile-Lys}$$

in which

    $X_8$      is His or Gln,

    $X_9$      is Gly or Ser,

    $X_{10}$      is Ser or Asp; and

L is a C-terminal part of from 390 to 400 amino acid residues which is at least 75 percent, preferably at least 80 percent, and more preferably at least 90 percent homologous to the 395 C-terminal amino acid residues in the Bacillus licheniformis 584 (ATCC 27811) alpha-amylase (Stephens et al., 1984, J.Bacteriol, 158:369-372).

Because of the relevance of Takkinen et al., supra, and Stephens et al., supra, in defining the amino acid sequences of the alpha amylases produced by B. amyloliquefaciens and B. licheniformis, portions of which sequences are contained within the chimeric amylases of the invention, these references are incorporated by reference herein in their entirety.

The amino acid sequence of the chimeric enzymes described and shown above may be modified by the substitution, deletion or addition of amino acid residues within the sequence which result in a silent change in the molecule so that the product retains its activity. Such amino acid substitutions may be made on the basis of similarity in polarity, charge, solubility, hydrophobicity, hydrophilicity and/or the amphipathic nature of the residues involved. For example, acidic amino acids (negatively charged at pH 6.0) include aspartic acid and glutamic acid; basic amino acids (positively charged at pH 6.0) include lysine and arginine; amino acids with uncharged polar head groups or nonpolar head groups having similar hydrophilic properties include the following: leucine, isoleucine, valine; glycine, alanine; asparagine, glutamine; serine, threonine; phenylalanine, tyrosine.

In another aspect the invention relates to processes for the production of the novel amylase of the first aspect above. According to this second aspect the amylases of the invention may be produced by the use of conventional genetic engineering techniques, such as gene splicing or by use of in vivo recombination to be described below, or by chemical synthetic techniques.

In a third aspect the invention relates to the use of the chimeric amylases in the liquefaction stage in the production of high DX syrups, especially in the jet cooking process mentioned above.

The chimeric alpha-amylases upon which the invention is based surprisingly demonstrate the excellent thermostability characteristics of alpha-amylase derived from B. licheniformis, but at the same time a reduced negative effect on the maximum obtainable DX without being inactivated prior to the saccharification. The invention is demonstrated herein, by way of examples, in which a segment of B. licheniformis alpha-amylase consisting of from about amino acid residue number 57 to about amino acid residue number 87, calculated from the N-terminal end of B. licheniformis alpha-amylase or, alternatively, the whole N-terminal segment thereof, is exchanged with the corresponding segment of B. amyloliquefaciens alpha-amylase. The residual activity of the chimeric alpha-amylase has at the most a negligible negative effect on the maximum DX obtainable by saccharification with glucoamylase-PROMOZYME while still retaining the excellent thermostability characteristic of B. licheniformis alpha-amylase.

## 3.1 DEFINITIONS

As used herein, the following terms shall have the meanings indicated:

DS = dry substance
DX = percentage by weight of dextrose (D-glucose).

## 4. BRIEF DESCRIPTION OF THE DRAWINGS

The invention will be described in further detail in the following specification and examples with reference to the appended drawing in which:

FIG. 1 shows gel-permeation chromatograms of alpha-amylases from B. licheniformis , B. amyloliquefaciens and an alpha-amylase according to the invention.
FIG. 2 shows the restriction map of plasmid pDN1822.
FIG. 3 shows the restriction map of plasmid pDN1850.
FIG. 4 shows the restriction map of plasmid PDN1864.

## 5. DETAILED DESCRIPTION OF THE INVENTION

As indicated above the invention relates to chimeric alpha-amylases of the general formula I

$$\textbf{(I)}$$

$$\textbf{Q-R-L}$$

in which Q, R, and L are defined as described in Section 3 supra. Preferred formulas are described in the subsections below.

## 5.1 AMINO ACID SEQUENCES OF PREFERRED EMBODIMENTS

A preferred alpha-amylase of the general formula I is one in which Q is an N-terminal part of the general formula Ib:

$$\textbf{(Ib)}$$

$$\overset{5}{} \qquad \overset{10}{} \qquad \overset{15}{}$$
$$X_1\text{-Asn-Gly-Thr-Leu-Met-Gln-Tyr-Phe-Glu-Trp-Tyr-}X_2\text{-Pro-Asn-Asp-}$$

$$\overset{20}{} \qquad \overset{25}{} \qquad \overset{30}{} \quad \overset{35}{}$$
$$\text{Gly-Gln-His-Trp-Lys-Arg-Leu-Gln-Asn-Asp-}X_3\text{-Leu-}X_4\text{-Gly-Ile-Thr-}$$

$$\overset{40}{} \qquad \overset{45}{} \qquad \overset{50}{}$$
$$\text{Ala-Val-Trp-Ile-Pro-Pro-Ala-Tyr-Lys-Gly-}X_5\text{-Ser-Gln-}X_6\text{-Asp-}X_7\text{-}$$

$$\overset{55}{}$$
$$\text{Gly-Tyr-Gly}$$

in which
$X_1$ is Ala-Asn-Leu or Val,
$X_2$ is Met or Thr,

X₃ is Ser-Ala-Tyr or Ala-Glu-His,
X₄ is Ala-Glu-His or Ser-Asp-Ile,
X₅ is Thr or Leu,
X₅ is Ala or Ser,
X₇ is Val or Asn; and
R and L are defined as previously in Section 3 supra.

In another preferred alpha-amylase of the general formula I, Q and R are defined as previously described, and L is a C-terminal part of the general formula Ic

(Ic)

      90                        95                      100

Ser-Leu-His-Ser-Arg-Asp-Ile-Asn-Val-Tyr-Gly-Asp-Val-

      105                      110                    115

Val-Ile-Asn-His-Lys-Gly-Gly-Ala-Asp-Ala-Thr-Glu-Asp-Val-Thr-

      120                      125                    130

Ala-Val-Glu-Val-Asp-Pro-Ala-Asp-Arg-Asn-Arg-Val-Ile-Ser-Gly-

      135                      140                    145

Glu-His-Arg-Ile-Lys-Ala-Trp-Thr-His-Phe-His-Phe-Pro-Gly-Arg-

      150                      155                    160

Gly-Ser-Thr-Tyr-Ser-Asp-Phe-Lys-Trp-His-Trp-Tyr-His-Phe-Asp-

      165                      170                    175

Gly-Thr-Asp-Trp-Asp-Glu-Ser-Arg-Lys-Leu-Asn-Arg-Ile-Tyr-Lys-

      180                      185                    190

Phe-Gln-Gly-Lys-Ala-Trp-Asp-Trp-Glu-Val-Ser-Asn-Glu-Asn-Gly-

```
                195                  200                  205
Asn-Tyr-Asp-Tyr-Leu-Met-Tyr-Ala-Asp-Ile-Asp-Tyr-Asp-His-Pro-
                210                  215                  220
Asp-Val-Ala-Ala-Glu-Ile-Lys-Arg-Trp-Gly-Thr-Trp-Tyr-Ala-Asn-
                225                  230                  235
Glu-Leu-Gln-Leu-Asp-Gly-Phe-Arg-Leu-Asp-Ala-Val-Lys-His-Ile-
                240                  245                  250
Lys-Phe-Ser-Phe-Leu-Arg-Asp-Trp-Val-Asn-His-Val-Arg-Glu-Lys-
                255                  260                  265
Thr-Gly-Lys-Glu-Met-Phe-Thr-Val-Ala-Glu-Tyr-Trp-Gln-Asn-Asp-
                270                  275                  280
Leu-Gly-Ala-Leu-Glu-Asn-Tyr-Leu-Asn-Lys-Thr-Asn-Phe-Asn-His-
                285                  290                  295
Ser-Val-Phe-Asp-Val-Pro-Leu-His-Tyr-Gln-Phe-His-Ala-Ala-Ser-
                300                  305                  319
Thr-Gln-Gly-Gly-Gly-Tyr-Asp-Met-Arg-Lys-Leu-Leu-Asn-Ser-Thr-
                315                  320                  325
Val-Val-Ser-Lys-His-Pro-Leu-Lys-Ala-Val-Thr-Phe-Val-Asp-Asn-
                330                  335                  340
His-Asp-Thr-Gln-Pro-Gly-Gln-Ser-Leu-Glu-Ser-Thr-Val-Gln-Thr-
                345                  350                  355
Trp-Phe-Lys-Pro-Leu-Ala-Tyr-Ala-Phe-Ile-Leu-Thr-Arg-Glu-Ser-
                360                  365                  370
Gly-Tyr-Pro-Gln-Val-Phe-Tyr-Gly-Asp-Met-Tyr-Gly-Thr-Lys-Gly-
                375                  380                  385
Asp-Ser-Gln-Arg-Glu-Ile-Pro-Ala-Leu-Lys-His-Lys-Ile-Glu-Pro-
                390                  395                  400
Ile-Leu-Lys-Ala-Arg-Lys-Gln-Tyr-Ala-Tyr-Gly-Ala-Gln-His-Asp-
                405                  410                  415
Tyr-Phe-Asp-His-His-Asp-Ile-Val-Gly-Trp-Thr-Arg-Glu-Gly-Asp-
                420                  425                  430
Ser-Ser-Val-Ala-Asn-Ser-Gly-Leu-Ala-Ala-Leu-Ile-Thr-Asp-Gly-
                435                  440                  445
Pro-Gly-Gly-Ala-Lys-Arg-Met-Tyr-Val-Gly-Arg-Gln-Asn-Ala-Gly-
```

6

<pre>
              450                    455                    460
Glu-Thr-Trp-His-Asp-Ile-Thr-Gly-Asn-Arg-Ser-Glu-Pro-Val-Val-
              465                    470                    475
 Ile-Asn-Ser-Glu-Gly-Trp-Gly-Glu-Phe-His-Val-Asn-Gly-Gly-Ser-
              480        483
Val-Ser-Ile-Tyr-Val-Gln-Arg
</pre>

In still another preferred alpha-amylase of the general formula I, Q has the general formula Ib and L is a C-terminal part of the general formula Ic, in which $X_1$ is Val, $X_2$ is Thr, $X_3$ is Ala-Glu-His, $X_4$ is Ser-Asp-Ile, $X_5$ is Leu, $X_5$ is Ser, and $X_7$ is Asn.

In yet another preferred alpha-amylase of the general formula I, Q has the general formula Ib in which $X_1$ is Val, $X_2$ is Thr, $X_3$ is Ala-Glu-His, $X_4$ is Ser-Asp-Ile, $X_5$ is Leu, $X_5$ is Ser, and $X_7$ is Asn; L is a C-terminal peptide residue of the general formula Ic; and amino acid residues $X_5$, $X_9$, and $X_{10}$ of R are Gln, Ser and Asp, respectively.

In yet another preferred alpha-amylase of the general formula I, Q has the general formula Ib in which Q, $X_1$ is Val, $X_2$ is Thr, $X_3$ is Ala-Glu-His, $X_4$ is Ser-Asp-Ile, $X_5$ is Leu, $X_5$ is Ser, and $X_7$ is Asn; L is a C-terminal part of the general formula Ic; and amino acid residues $X_5$ $X_9$ and $X_{10}$ of R are His, Gly and Ser, respectively.

## 5.2 METHODS FOR PRODUCING THE CHIMERIC AMYLASES

The amylases of the invention are chimeric enzymes and may in accordance with the second aspect of the invention be produced in a number of ways as described below.

Naturally occurring enzymes may be genetically modified by random or site directed mutagenesis. Alternatively, part of one enzyme may be replaced by a part of another to obtain a chimeric enzyme. This replacement can be achieved either by conventional in vitro gene splicing techniques or by in vivo recombination or by combinations of both techniques. When using conventional in vitro gene splicing techniques, a desired portion of the alpha-amylase gene coding sequence may be deleted using appropriate site-specific restriction enzymes; the deleted portion of the coding sequence may then be replaced by the insertion of a desired portion of a different alpha-amylase coding sequence so that a chimeric nucleotide sequence encoding a new alpha-amylase is produced.

The in vivo recombinantion techniques depend on the fact that different DNA segments with highly homologous regions (identity of DNA sequence) may recombine, i.e. break and exchange DNA, and establish new bonds in the homologous regions. Accordingly, when the coding sequences for two different but homologous amylase enzymes are used to transform a host cell, recombination of homologous sequences in vivo will result in the production of chimeric gene sequences. Translation of these coding sequences by the host cell will result in production of a chimeric amylase gene product.

The alpha-amylase genes from Bacillus licheniformis (herein designated amyL) and from Bacillus amyloliquefaciens (herein designated amyQ) are approximately 70 percent homologous at the DNA level and suitable for hybrid formation by in vivo gene splicing.

In an alternate embodiment, the chimeric enzyme may be synthesized by standard chemical methods known in the art. For example, see Hunkapiller et al., 1984, Nature 310:105-111. Accordingly, peptides having the amino acid sequences described supra may be synthesized in whole or in part and joined to form the chimeric enzymes of the invention.

## 5.3 USES OF THE CHIMERIC AMYLASES

According to its third aspect the invention relates to the use of the novel alpha-amylases in the liquefaction stage in the overall enzymatic conversion of starch into high DX syrups.

As indicated previously residual activity from the use of the thermostable alpha-amylase from B. licheniformis in the liquefaction stage entails a negative effect on maximum obtainable D-glucose yield in the saccharification stage when using A. niger glucoamylase and B. acidopullulyticus pullulanase.

The reason for this negative effect is not fully understood, but it is assumed that B. licheniformis alpha-amylase generates "limit dextrins" which are poor substrates for B. acidopullulyticus pullulanase, by hydrolyzing 1, 4-alpha-glucosidic linkages close to the branch-points in amylopectin. These limit dextrins which contain too few glucose units in one or more of the side chains will be less susceptible to B. acidopullulyticus pullulanase attack.

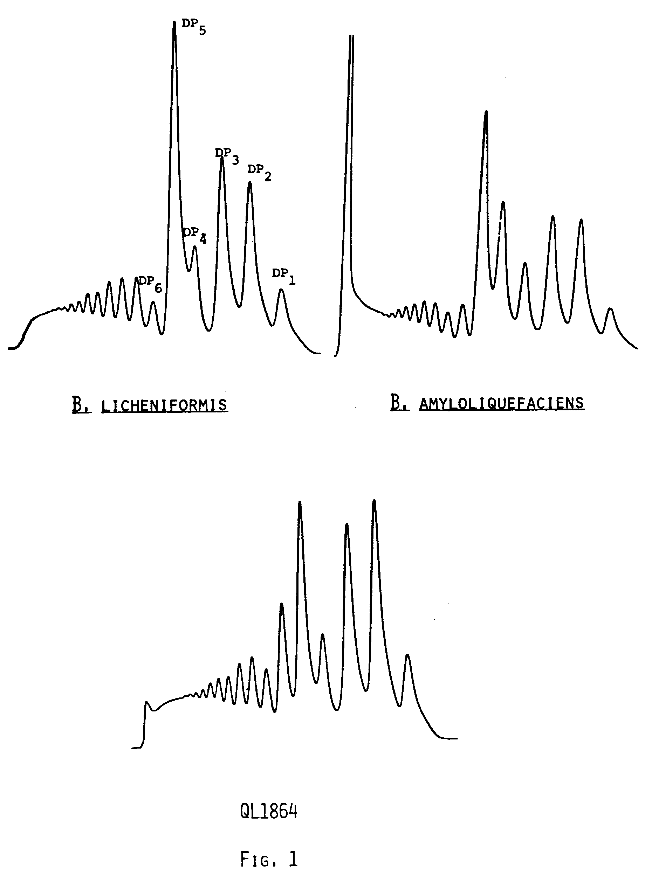

In FIG. 1 the action patterns for B. licheniformis alpha-amylase, B. amyloliquefaciens alpha-amylase, and the hybrid QL1864 alpha-amylase on amylopectin are indicated by the gel-permeation chromatograms taken from amylopectin digests after 48 hours.

From the figure it is seen that the action pattern of B. licheniformis alpha-amylase on amylopectin is different from that of B. amyloliquefaciens alpha-amylase. The B. licheniformis enzyme produces mainly $DP_6$, $DP_5$ and $DP_3$ initially. On prolonged hydrolysis the $DP_6$ fraction is hydrolyzed further, and the major components are $DP_5$, $DP_3$, and $DP_2$. When B. amyloliquefaciens alpha-amylase is used the major components are $DP_6$.

The action pattern of the alpha-amylases of the invention as exemplified by the QL1864 alpha-amylase on amylopectin is distinctly different from both naturally occurring alpha-amylases, and as shown below, this changed action pattern surprisingly has resulted in the removal of the negative effect from B. licheniformis alpha-amylase on the D-glucose yield, while retaining the thermostability.

Accordingly it has been found that the alpha-amylases of the invention are very efficiently used for the liquefaction of starch.

## 6. EXAMPLE: CHIMERIC AMYLASE QL1864

The subsections below describe the production and characterization of the chimeric alpha-amylase QL1864.

### 6.1. CONSTRUCTION OF HYBRID QL1864

By conventional techniques, amyL and amyQ were cloned in B. subtilis. The restriction enzyme map of the two genes were in agreement with published DNA sequences for the genes for B. licheniformis amylase (amyL) (Stephens et al. 1986, J. Bacteriol. 158: 369 (1984)) and B. amyloliquefaciens amylase (amyQ) (Takkinen et al., 1983, J. Biol. Chem 258: 1007) 1983), respectively.

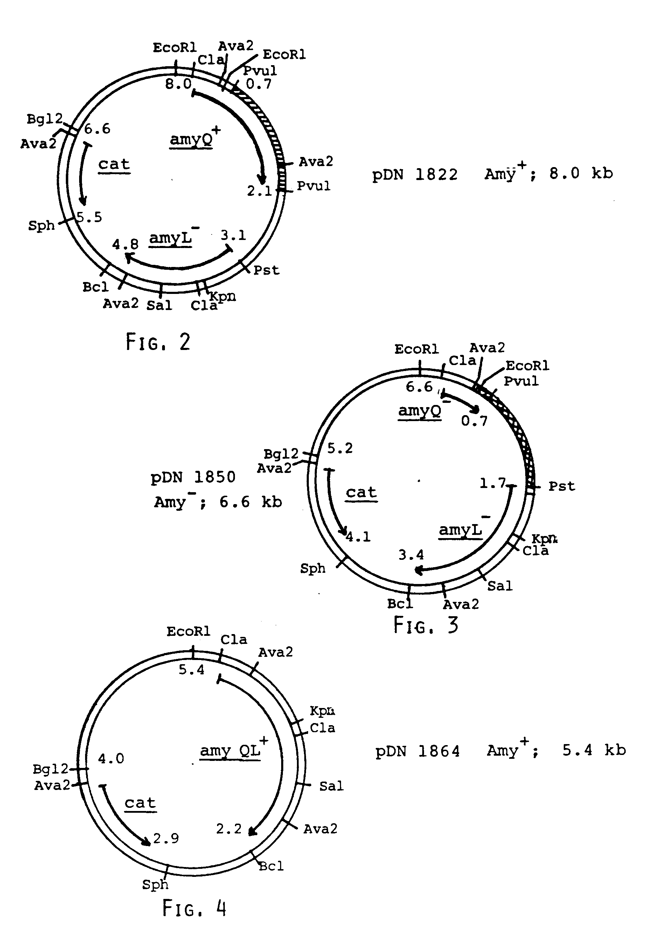

amyQ (amyQ+) and a C-terminal part of amyL (amyL- were placed in parallel on plasmid pDN1822. This is a B. subtilis plasmid derived from cloning vector pUB110 and harbouring the chloramphenicol resistance (Cam$^R$) gene (cat gene) of cloning vector pC194. The restriction map of pDN1822 is shown in FIG. 2, where the genes are indicated by arrows. The C-terminal part of amyQ on pDN1822 was then deleted by excision of a PvuI-PvuI fragment, which is shown hatched in FIG. 2, to obtain plasmid pDN1850 (FIG. 3). pDN1850 is amylase negative (Amy-) but harbors a N-terminal-part of amyQ and a C-terminal part of amyL. However, with a frequency of about $10^{-4}$, recombination between amyQ and amyL occurs resulting in the plasmids harbouring a hybrid QL amylase gene (amyQL+) and of an amylase positive phenotype (Amy+).

Transformation with a plasmid preparation of pDN1850 into a plasmid free B. subtilis recipient selecting for Cam$^R$ on starch containing agar plates resulted in about 1:10$^4$ transformants producing an active amylase. These transformants were surrounded by a halo of degraded starch which could be identified by iodine vapour. These Amy$^+$ transformants harboured a QL hybrid amylase gene on the plasmid. From these transformants the plasmids pDN1851 to pDN1865 were isolated, and it was found that transformants containing plasmids pDN1851, pDN1858 to pDN1862 and pDN1864 produced alpha-amylases that fulfill the objects of the invention. By restriction enzyme mapping of plasmid pDN1864, the amyQ/L1864 gene was characterized (FIG. 4) and shown to harbor an AvaII site from amyQ, but not the nearby EcoRI site from amyQ. Hence, recombination between amyQ and amyL as indicated by the cross-hatched area in FIG. 3 took place between the codons coding for amino acid No. 58 and No. 67 in the B. licheniformis alpha-amylase. B. subtilis QL1864 is therefore producing a chimeric amylase composed of about 1/6 amyQ amylase at the N-terminal end and about 5/6 amyL amylase at the C-terminal end.

### 6.2 ANALYSIS OF CHIMERIC AMYLASE PRODUCED BY QL1864

In the following tests the enzyme units used are defined as indicated below:

One NU (NOVO Unit) of alpha-amylase activity is the amount of enzyme which breaks down 5.26 mg of dissolved starch per hour at 37°C, pH 5.6 and 0.0043 M of Ca$^{++}$ over a 7-20 minute reaction time.

One AG unit of glucoamylase activity is the amount of enzyme which hydrolyzes one micromole of maltose per minute at 25°C and pH 4.3.

One pullulanase unit (PUN) is defined as the amount of enzyme which under standard conditions (temperature 40°C and pH 5.0) hydrolyzes pullulan at a rate corresponding to the formation of reducing groups equivalent to 1 μmole of glucose per minute.

## 6.2.1. SACCHARIFICATION TEST OF CHIMERIC AMYLASE

As explained above it has been found that the presence of a residual B. licheniformis alpha-amylase activity originating from the liquefaction stage has a negative effect on maximum D-glucose yield in the saccharification stage when B. acidopullulyticus pullulanase and A. niger glucoamylase are used in combination.

In order to evaluate the influence of a residual activity from the chimeric alpha-amylases of the invention on the saccharification stage they were compared to the B. licheniformis alpha-amylase in the following way:

Substrates for saccharification were prepared by redissolving a DE 8 spray-dried maltodextrin (APS 840964A) in deionized water the making up to approximately 30% DS (dry substance). Saccharification experiments were carried out in standard 500 ml laboratory batch reactions.

pH's were measured at saccharification temperature with the pH electrode and pH meter calibrated and adjusted in buffer at 60°C.

The following standard conditions were used:

| Substrate concentration | 28.2% (initial) | 30.8% (final) |
|---|---|---|
| Temperature | 60°C | |
| pH (initial, at 60°C) | 4.6 | |
| Enzyme dossage: | | |
| glucoamylase | 0.15 AG/g DS | |
| pullulanase | 0.33 PUN/g DS | |
| alpha-amylase | 60 NU/g DS | |

The results of the tests are presented in Table I

## TABLE I
## SACCHARIFICATION TEST OF CHIMERIC AMYLASE

| Alpha-Amylase | Reaction Conditions time (h) | pH | %DP$_1$ | %DP$_2$ | %DP$_3$ | %DP$_4$ |
|---|---|---|---|---|---|---|
| None | 24 | 4.5 | 92.8 | 2.5 | 1.1 | 3.6 |
| (Control) | 48 | 4.4 | 96.7 | 1.8 | 0.7 | 0.8 |
| | 72 | 4.4 | 96.8 | 2.0 | 0.6 | 0.6 |
| | 96 | 4.4 | 96.8 | 2.2 | 0.5 | 0.5 |
| B. licheniformis | 24 | 4.5 | 92.4 | 2.5 | 2.4 | 2.7 |
| | 48 | 4.5 | 95.9 | 1.8 | 1.5 | 0.9 |
| | 72 | 4.4 | 96.2 | 2.0 | 1.1 | 0.7 |
| | 96 | 4.4 | 96.4 | 2.1 | 0.9 | 0.6 |
| QL 1864 | 24 | 4.6 | 92.1 | 2.8 | 1.9 | 3.2 |
| | 48 | 4.5 | 96.3 | 1.7 | 1.2 | 0.9 |
| | 72 | 4.5 | 96.5 | 2.0 | 0.9 | 0.7 |
| | 96 | 4.5 | 96.6 | 2.1 | 0.8 | 0.6 |

From the results shown in Table I it is seen that although the presence of QL 1864 alpha-amylase slightly reduced the maximum obtainable DX (in comparison to the control), it represents a significant improvement over the B. licheniformis alpha-amylase.

6.2.2 THERMOACTIVATION OF CHIMERIC AMYLASE

In order to evaluate the thermoactivation of the chimeric alpha-amylases produced by the transformed strains the chimeric alpha-amylases were submitted to the following test:

Substrate: Phadebas tables (Phadebas® amylase test, Pharmacia Diagnostics, Sweden) a cross-linked blue coloured starch polymer insoluble in water.

Buffer: 0.1 M phosphate, pH 6.1, and TRIS buffer pH 9.5.

Enzyme: alpha-Amylase diluted to 1-2 NU/ml in 0.09 M CaCl$_2$, pH 6.1.

Temperatures: 37°C and 85°C

1 ml alpha-amylase dilution was thoroughly mixed with 5 ml buffer and incubated in a water bath at the desired temperature prior to the addition of one Phadebas table.

The test tube was shaken for 15 seconds on a whirl mixer before it is placed in the water bath again.

After exactly 15 minutes the reaction was stopped by the addition of 1 ml 1 M NaOH. After mixing the mixture was filtered through a 9 cm Whatman® GF/A of FG/C filter.

The optical density of the filtrate was measured at a wavelength of 620 nm, and was found to be linearly related to the activity of alpha-amylase added.

The results are presented in Table II below together with values from tests with pure B. licheniformis and B. amyloliquefaciens alpha-amylases.

## TABLE II
## THERMOACTIVATION OF CHIMERIC AMYLASE

| Alpha-Amylase | Phadebas 37°C pH 6.1:pH 9.5 | Phadebas pH 6.1 75°C:37°C |
|---|---|---|
| B. licheniformis (control) | 0.4 | 3.7 |
| QL1864 | 2.5 | 2.5 |
| QL1861 | 2.2 | 2.2 |
| QL1851 | 2.1 | 2.1 |
| QL1862 | 2.0 | 2.0 |
| QL1858 | 2.0 | 2.0 |
| B. amyloliquefaciens (control) | 8.7 | 0.01 |

The data presented in Table II demonstrate that the chimeric alpha-amylases of the invention are as thermoactivated as the B. licheniformis alpha-amylase, and less sensitive to alkaline pH than the B. amyloliquefaciens alpha-amylase.

6.2.3. THERMOSTABILITY OF CHIMERIC AMYLASE

In order to evaluate the stability of the alpha-amylases of the invention the following steel tube tests were performed:

A DE 7 maltodextrin redissolved in deionized water was used as substrate under the following conditions:

Substrate: 32 - 33 percent
alpha-amylase dosage: 120 NU/g maltodextrin
Temperature: 105°C
pH: 5.5
Calcium content: 60 ppm

In each test 5 steel tubes containing the above reaction mixture were placed in an oil bath at 105°C and taken out after 10, 20, 30, 40, and 60 minutes, respectively, and the residual alpha-amylase activity measured by the Phadebas method described above. The half life, $T_{1/2}$, is calculated by linear regression of log (residual activity) versus time. The results are shown in Table III below.

TABLE III

| THERMOSTABILITY OF CHIMERIC AMYLASES | |
|---|---|
| Alpha-Amylase | $T_{1/2}$ minutes |
| B. amyloliquefaciens (control) | 5 |
| QL 1851 | 22 |
| QL 1858 | 25 |
| QL 1861 | 18 |
| QL 1862 | 22 |
| QL 1864 | 24 |
| B. licheniformis (control) | 23 |

11

From the results shown in Table III it is clearly seen that the hybrid alpha-amylases of the invention have retained the stability of the B. licheniformis alpha-amylase.

**Claims**

**Claims for the following Contracting States : BE, CH, DE, FR, GB, IT, LI, LU, NL, SE**

1. A chimeric alpha-amylase, which is thermostable and exhibits a reduced negative effect on the use of A. niger glucoamylase and B. acidopullulyticus pullulanase for the saccharification of starch, having the general formula I

$$\textbf{(I)}$$
$$\textbf{Q-R-L}$$

in which Q comprises a N-terminal part of from 55 to 60 amino acid residues which is at least 75% homologous to the 55 N-terminal amino acid residues in the Bacillus amyloliquefaciens alpha-amylase as described in Takkinen et al., J. Biol. Chem. 258 (1983) 1007-1013;

R comprises a part of the general formula Ia

$$\textbf{(Ia)}$$

$$\overset{58}{\text{Pro}}-\text{Tyr}-\text{Asp}-\overset{60}{\text{Leu}}-\text{Tyr}-\text{Asp}-\text{Leu}-\text{Gly}-\text{Glu}-\text{Phe}-\overset{70}{X_8}-\text{Gln}-\text{Lys}-$$

$$\text{Gly}-\text{Thr}-\text{Val}-\text{Arg}-\text{Thr}-\text{Lys}-\text{Tyr}-\text{Gly}-\text{Thr}-\text{Lys}-\overset{80}{X_9}-\text{Glu}-\text{Leu}$$

$$\overset{88}{\text{Gln}}-X_{10}-\text{Ala}-\text{Ile}-\text{Lys}$$

in which

$X_8$        comprises His or Gln,

$X_9$        comprises Gly or Ser,

$X_{10}$      comprises Ser or Asp; and

L comprises a C-terminal part of from 390 to 400 amino acid residues which is at least 75% homologous to the 395 C-terminal amino acid residues in the Bacillus licheniformis 584 (ATCC 27811) alpha-amylase.

2. The chimeric alpha-amylase according to claim 1, in which

$X_8$        comprises His,

$X_9$        comprises Gly, and

$X_{10}$      comprises Ser.

3. The chimeric alpha-amylase according to claim 1, in which

$X_8$        comprises Gln,

$X_9$        comprises Ser, and

$X_{10}$      comprises Asp.

4. The chimeric alpha-amylase according to claim 1, in which the homologies are at least 80 percent.

5. The chimeric alpha-amylase according to claim 1, in which the homologies are at least 90 percent.

6. The chimeric alpha-amylase according to claim 1, in which Q comprises an N-terminal part of the general formula Ib

$$\text{(Ib)}$$

$$X_1\text{-Asn-Gly-Thr-Leu-Met-Gln-Tyr-Phe-Glu-Trp-Tyr-}X_2\text{-Pro-Asn-}$$

$$\text{Asp-Gly-Gln-His-Trp-Lys-Arg-Leu-Gln-Asn-Asp-}X_3\text{-Leu-}X_4\text{-Gly-}$$

$$\text{Ile-Thr-Ala-Val-Trp-Ile-Pro-Pro-Ala-Tyr-Lys-Gly-}X_5\text{-Ser-Gln-}$$

$$X_6\text{-Asp-}X_7\text{-Gly-Tyr-Gly;}$$

in which

| | |
|---|---|
| $X_1$ | comprises Ala-Asn-Leu or Val, |
| $X_2$ | comprises Met or Thr, |
| $X_3$ | comprises Ser-Ala-Tyr or Ala-Glu-His, |
| $X_4$ | comprises Ala-Gly-His or Ser-Asp-Ile, |
| $X_5$ | comprises Thr or Leu, |
| $X_5$ | comprises Ala or Ser, and |
| $X_7$ | comprises Val or Asn. |

7. The chimeric alpha-amylase according to claim 6, in which

| | |
|---|---|
| $X_1$ | comprises Val, |
| $X_2$ | comprises Thr, |
| $X_3$ | comprises Ala-Glu-His, |
| $X_4$ | comprises Ser-Asp-Ile, |
| $X_5$ | comprises Leu, |
| $X_5$ | comprises Ser, and |
| $X_7$ | comprises Asn. |

8. The alpha-amylase according to claim 4, 5, 6, or 7, in which L comprises a C-terminal part of the general formula Ic

## (Ic)

```
              90                    95                   100
      Ser-Leu-His-Ser-Arg-Asp-Ile-Asn-Val-Tyr-Gly-Asp-Val


             105                   110                   115
  Val-Ile-Asn-His-Lys-Gly-Gly-Ala-Asp-Ala-Thr-Glu-Asp-Val-Thr-


             120                   125                   130
  Ala-Val-Glu-Val-Asp-Pro-Ala-Asp-Arg-Asn-Arg-Val-Ile-Ser-Gly-


             135                   140                   145
  Glu-His-Arg-Ile-Lys-Ala-Trp-Thr-His-Phe-His-Phe-Pro-Gly-Arg-


             150                   155                   160
  Gly-Ser-Thr-Tyr-Ser-Asp-Phe-Lys-Trp-His-Trp-Tyr-His-Phe-Asp-


             165                   170                   175
  Gly-Thr-Asp-Trp-Asp-Glu-Ser-Arg-Lys-Leu-Asn-Arg-Ile-Tyr-Lys-


             180                   185                   190
  Phe-Gln-Gly-Lys-Ala-Trp-Asp-Trp-Glu-Val-Ser-Asn-Glu-Asn-Gly-


             195                   200                   205
  Asn-Tyr-Asp-Tyr-Leu-Met-Tyr-Ala-Asp-Ile-Asp-Tyr-Asp-His-Pro-


             210                   215                   220
  Asp-Val-Ala-Ala-Glu-Ile-Lys-Arg-Trp-Gly-Thr-Trp-Tyr-Ala-Asn-


             225                   230                   235
  Glu-Leu-Gln-Leu-Asp-Gly-Phe-Arg-Leu-Asp-Ala-Val-Lys-His-Ile-


             240                   245                   250
  Lys-Phe-Ser-Phe-Leu-Arg-Asp-Trp-Val-Asn-His-Val-Arg-Glu-Lys-


             255                   260                   265
  Thr-Gly-Lys-Glu-Met-Phe-Thr-Val-Ala-Glu-Tyr-Trp-Gln-Asn-Asp-


             270                   275                   280
  Leu-Gly-Ala-Leu-Glu-Asn-Tyr-Leu-Asn-Lys-Thr-Asn-Phe-Asn-His-


             285                   290                   285
  Ser-Val-Phe-Asp-Val-Pro-Leu-His-Tyr-Gln-Phe-His-Ala-Ala-Ser-


             300                   305                   310
  Thr-Gln-Gly-Gly-Gly-Tyr-Asp-Met-Arg-Lys-Leu-Leu-Asn-Ser-Thr-
```

```
                315                 320                 325
Val-Val-Ser-Lys-His-Pro-Leu-Lys-Ala-Val-Thr-Phe-Val-Asp-Asn-

                330                 335                 340
His-Asp-Thr-Gln-Pro-Gly-Gln-Ser-Leu-Glu-Ser-Thr-Val-Gln-Thr-

                345                 350                 355
Trp-Phe-Lys-Pro-Leu-Ala-Tyr-Ala-Phe-Ile-Leu-Thr-Arg-Glu-Ser-

                360                 365                 370
Gly-Tyr-Pro-Gln-Val-Phe-Tyr-Gly-Asp-Met-Tyr-Gly-Thr-Lys-Gly-

                375                 380                 385
Asp-Ser-Gln-Arg-Glu-Ile-Pro-Ala-Leu-Lys-His-Lys-Ile-Glu-Pro-

                390                 395                 400
Ile-Leu-Lys-Ala-Arg-Lys-Gln-Tyr-Ala-Tyr-Gly-Ala-Gln-His-Asp-

                405                 410                 415
Tyr-Phe-Asp-His-His-Asp-Ile-Val-Gly-Trp-Thr-Arg-Glu-Gly-Asp-

                420                 425                 430
Ser-Ser-Val-Ala-Asn-Ser-Gly-Leu-Ala-Ala-Leu-Ile-Thr-Asp-Gly-

                435                 440                 445
Pro-Gly-Gly-Ala-Lys-Arg-Met-Tyr-Val-Gly-Arg-Gln-Asn-Ala-Gly-

                450                 455                 460
Glu-Thr-Trp-His-Asp-Ile-Thr-Gly-Asn-Arg-Ser-Glu-Pro-Val-Val-

                465                 470                 475
Ile-Asn-Ser-Glu-Gly-Trp-Gly-Glu-Phe-His-Val-Asn-Gly-Gly-Ser-

                480         483
Val-Ser-Ile-Tyr-Val-Gln-Arg.
```

**9.** A process for the production of a chimeric alpha-amylase comprising:

(a) recombining in vivo the N-terminal coding region of the alpha-amylase gene of B. amyloliquefaciens with the C-terminal coding region of the alpha-amylase gene of B. licheniformis to form recombinants;

(b) selecting the recombinants that produce a chimeric alpha-amylase that is thermostable and exhibits a reduced negative effect on the use of A. niger glucoamylase and B. acidopullulyticus pullulanase for the saccharification of starch;

(c) culturing the selected recombinants in an appropriate growth medium, and

(d) recovering the chimeric alpha-amylase from the culture.

**10.** A process for converting starch into high dextrose syrup, comprising:

(a) reacting the starch with the chimeric alpha-amylase of claim 1, 2, 3, 4, 5, 6, or 7 to form oligosaccharides; and

(b) reacting the oligosaccharides formed in step (a) with a glucoamylase to form dextrose.

**11.** A process for converting starch into high dextrose syrup, comprising:

(a) reacting the starch with the chimeric alpha-amylase of claim 8 to form oligosaccharides; and

(b) reacting the oligosaccharides formed in step (a) with a glucoamylase to form dextrose.

**Claims for the following Contracting States : AT, ES, GR**

1. A process for the production of a chimeric alpha-amylase comprising:
   (a) recombining in vivo the N-terminal coding region of the alpha-amylase gene of B. amyloliquefaciens with the C-terminal coding region of the alpha-amylase gene of B. licheniformis to form recombinants;
   (b) selecting the recombinants that produce a chimeric alpha-amylase that is thermostable and exhibits a reduced negative effect on the use of A. niger glucoamylase and B. acidopullulyticus pullulanase for the saccharification of starch;
   (c) culturing the selected recombinants in an appropriate growth medium, and
   (d) recovering the chimeric alpha-amylase from the culture.

2. A process for converting starch into high dextrose syrup, comprising:
   (a) reacting the starch with a chimeric alpha-amylase, which is thermostable and exhibits a reduced negative effect on the use of A. niger glucoamylase and B. acidopullulyticus pullulanase for the saccharification of starch, having the general formula I

$$\textbf{(I)}$$

$$\textbf{Q-R-L}$$

in which Q comprises a N-terminal part of from 55 to 60 amino acid residues which is at least 75% homologous to the 55 N-terminal amino acid residues in the Bacillus amyloliquefaciens alpha-amylase as described in Takkinen et al., J. Biol. Chem. 258 (1983) 1007-1013;
   R comprises a part of the general formula Ia

$$\textbf{(Ia)}$$

$$\overset{\text{58}}{\text{Pro-Tyr-Asp-}}\overset{\text{60}}{\text{Leu-Tyr}}\text{-Asp-Leu-Gly-Glu-Phe-}\underset{\text{8}}{X}\text{-Gln-}\overset{\text{70}}{\text{Lys-}}$$

$$\text{Gly-Thr-Val-Arg-Thr-Lys-Tyr-Gly-Thr-}\overset{\text{80}}{\text{Lys-}}X_9\text{-Glu-Leu}$$

$$\text{Gln-}\underset{\text{10}}{X}\text{-}\overset{\text{88}}{\text{Ala-Ile-Lys}}$$

in which
   $X_8$ comprises His or Gln,
   $X_9$ comprises Gly or Ser,
   $X_{10}$ comprises Ser or Asp; and
   L comprises a C-terminal part of from 390 to 400 amino acid residues which is at least 75% homologous to the 395 C-terminal amino acid residues in the Bacillus licheniformis 584 (ATCC 27811) alpha-amylase, to form oligosaccharides: and
   (b) reacting the oligosaccharides formed in step (a) with a glucoamylase to form dextrose.

3. A process according to Claim 2, wherein, in the chimeric alpha-amylase,
   $X_8$ comprises His,
   $X_9$ comprises Gly, and
   $X_{10}$ comprises Ser.

4. A process according to Claim 2, wherein, in the chimeric alpha-amylase
   $X_8$ comprises Gln,
   $X_9$ comprises Ser, and
   $X_{10}$ comprises Asp.

5. A process according to Claim 2, wherein, in the chimeric alpha-amylase, the homologies are at least 80 percent.

6. A process according to Claim 2, wherein, in the chimeric alpha-amylase the homologies are at least 90 percent.

7. A process according to Claim 2, wherein, in the chimeric alpha-amylase, Q comprises an N-terminal part of the general formula Ib

$$(Ib)$$

$$5 \qquad\qquad 10 \qquad\qquad 15$$

$$X_1\text{-Asn-Gly-Thr-Leu-Met-Gln-Tyr-Phe-Glu-Trp-Tyr-}X_2\text{-Pro-Asn-}$$

$$20 \qquad\qquad 25 \qquad\qquad 30 \quad\; 35$$

$$\text{Asp-Gly-Gln-His-Trp-Lys-Arg-Leu-Gln-Asn-Asp-}X_3\text{-Leu-}X_4\text{-Gly-}$$

$$40 \qquad\qquad 45 \qquad\qquad 50$$

$$\text{Ile-Thr-Ala-Val-Trp-Ile-Pro-Pro-Ala-Tyr-Lys-Gly-}X_5\text{-Ser-Gln-}$$

$$55$$

$$X_6\text{-Asp-}X_7\text{-Gly-Tyr-Gly;}$$

in which
$X_1$     comprises Ala-Asn-Leu or Val,
$X_2$     comprises Met or Thr,
$X_3$     comprises Ser-Ala-Tyr or Ala-Glu-His,
$X_4$     comprises Ala-Gly-His or Ser-Asp-Ile,
$X_5$     comprises Thr or Leu,
$X_5$     comprises Ala or Ser, and
$X_7$     comprises Val or Asn.

8. A process according to Claim 7, wherein, in the chimeric alpha-amylase,
$X_1$     comprises Val,
$X_2$     comprises Thr,
$X_3$     comprises Ala-Glu-His,
$X_4$     comprises Ser-Asp-Ile,
$X_5$     comprises Leu,
$X_5$     comprises Ser, and
$X_7$     comprises Asn.

9. A process for converting starch into high dextrose syrup, comprising:
(a) reacting the starch with a chimeric alpha-amylase to form oligosaccharides; and
(b) reacting the oligosaccharides formed in step (a) with a glucoamylase to form dextrose, the chimeric alpha-amylase being as defined in Claim 5, 6, 7 or 8 and in which L comprises a C-terminal part of the general formula Ic

## (Ic)

90                              95                             100
Ser-Leu-His-Ser-Arg-Asp-Ile-Asn-Val-Tyr-Gly-Asp-Val

105                            110                             115
Val-Ile-Asn-His-Lys-Gly-Gly-Ala-Asp-Ala-Thr-Glu-Asp-Val-Thr-

120                            125                             130
Ala-Val-Glu-Val-Asp-Pro-Ala-Asp-Arg-Asn-Arg-Val-Ile-Ser-Gly-

135                            140                             145
Glu-His-Arg-Ile-Lys-Ala-Trp-Thr-His-Phe-His-Phe-Pro-Gly-Arg-

150                            155                             160
Gly-Ser-Thr-Tyr-Ser-Asp-Phe-Lys-Trp-His-Trp-Tyr-His-Phe-Asp-

165                            170                             175
Gly-Thr-Asp-Trp-Asp-Glu-Ser-Arg-Lys-Leu-Asn-Arg-Ile-Tyr-Lys-

180                            185                             190
Phe-Gln-Gly-Lys-Ala-Trp-Asp-Trp-Glu-Val-Ser-Asn-Glu-Asn-Gly-

195                            200                             205
Asn-Tyr-Asp-Tyr-Leu-Met-Tyr-Ala-Asp-Ile-Asp-Tyr-Asp-His-Pro-

210                            215                             220
Asp-Val-Ala-Ala-Glu-Ile-Lys-Arg-Trp-Gly-Thr-Trp-Tyr-Ala-Asn-

225                            230                             235
Glu-Leu-Gln-Leu-Asp-Gly-Phe-Arg-Leu-Asp-Ala-Val-Lys-His-Ile-

240                            245                             250
Lys-Phe-Ser-Phe-Leu-Arg-Asp-Trp-Val-Asn-His-Val-Arg-Glu-Lys-

255                            260                             265
Thr-Gly-Lys-Glu-Met-Phe-Thr-Val-Ala-Glu-Tyr-Trp-Gln-Asn-Asp-

270                            275                             280
Leu-Gly-Ala-Leu-Glu-Asn-Tyr-Leu-Asn-Lys-Thr-Asn-Phe-Asn-His-

285                            290                             285
Ser-Val-Phe-Asp-Val-Pro-Leu-His-Tyr-Gln-Phe-His-Ala-Ala-Ser-

300                            305                             310
Thr-Gln-Gly-Gly-Gly-Tyr-Asp-Met-Arg-Lys-Leu-Leu-Asn-Ser-Thr-

```
            315                   320                   325
Val-Val-Ser-Lys-His-Pro-Leu-Lys-Ala-Val-Thr-Phe-Val-Asp-Asn-

            330                   335                   340
His-Asp-Thr-Gln-Pro-Gly-Gln-Ser-Leu-Glu-Ser-Thr-Val-Gln-Thr-

            345                   350                   355
Trp-Phe-Lys-Pro-Leu-Ala-Tyr-Ala-Phe-Ile-Leu-Thr-Arg-Glu-Ser-

            360                   365                   370
Gly-Tyr-Pro-Gln-Val-Phe-Tyr-Gly-Asp-Met-Tyr-Gly-Thr-Lys-Gly-

            375                   380                   385
Asp-Ser-Gln-Arg-Glu-Ile-Pro-Ala-Leu-Lys-His-Lys-Ile-Glu-Pro-

            390                   395                   400
Ile-Leu-Lys-Ala-Arg-Lys-Gln-Tyr-Ala-Tyr-Gly-Ala-Gln-His-Asp-

            405                   410                   415
Tyr-Phe-Asp-His-His-Asp-Ile-Val-Gly-Trp-Thr-Arg-Glu-Gly-Asp-

            420                   425                   430
Ser-Ser-Val-Ala-Asn-Ser-Gly-Leu-Ala-Ala-Leu-Ile-Thr-Asp-Gly-

            435                   440                   445
Pro-Gly-Gly-Ala-Lys-Arg-Met-Tyr-Val-Gly-Arg-Gln-Asn-Ala-Gly-

            450                   455                   460
Glu-Thr-Trp-His-Asp-Ile-Thr-Gly-Asn-Arg-Ser-Glu-Pro-Val-Val-

            465                   470                   475
Ile-Asn-Ser-Glu-Gly-Trp-Gly-Glu-Phe-His-Val-Asn-Gly-Gly-Ser-

            480        483
Val-Ser-Ile-Tyr-Val-Gln-Arg.
```

**Patentansprüche**

**Patentansprüche für folgende Vertragsstaaten : BE, CH, DE, FR, GB, IT, LI, LU, NL, SE**

1.  Chimäre Alpha-Amylase, die hitzestabil ist und eine verringerte negative Wirkung auf die Verwendung von Glucoamylase aus A. niger und Pullulanase aus B. acidopulluliticus zur Verzuckerung von Stärke zeigt, mit der allgemeinen Formel I

$$(I)$$

$$Q-R-L,$$

in der Q eine N-terminalen Teil mit von 55 bis 60 Aminosäureresten umfaßt, der wenigstens 75% homolog zu den 55 N-terminalen Aminosäureresten in der Alpha-Amylase aus Bacillus amyloliquefaciens ist, wie in Takkinen et al., J. Biol. Chem. 258 (1983), 1007-1013 beschrieben;
R einen Teil der allgemeinen Formel Ia umfaßt

(Ia)

Pro-Tyr-Asp-Leu-Tyr-Asp-Leu-Gly-Glu-Phe-$X_8$-Gln-Lys-

Gly-Thr-Val-Arg-Thr-Lys-Tyr-Gly-Thr-Lys-$X_9$-Glu-Leu

Gln-$X_{10}$-Ala-Ile-Lys

in der

    $X_8$      His oder Gln umfaßt,

    $X_9$      Gly oder Ser umfaßt,

    $X_{10}$    Ser oder Asp umfaßt; und

    L einen C-terminalen Teil mit von 390 bis 400 Aminosäureresten umfaßt, der wenigstens 75% homolog zu den 395 C-terminalen Aminosäureresten in der Alpha-Amylase aus Bacillus licheniformis 584 (ATCC 27811) ist.

    **2.**   Chimäre Alpha-Amylase nach Anspruch 1, in der

        $X_8$      His umfaßt,

        $X_9$      Gly umfaßt, und

        $X_{10}$    Ser umfaßt.

    **3.**   Chimäre Alpha-Amylase nach Anspruch 1, in der

        $X_8$      Gln umfaßt,

        $X_9$      Ser umfaßt und

        $X_{10}$    Asp umfaßt.

    **4.**   Chimäre Alpha-Amylase nach Anspruch 1, in der die Homologien wenigstens 80 Prozent sind.

    **5.**   Chimäre Alpha-Amylase nach Anspruch 1, in der die Homologien wenigstens 90 Prozent sind.

    **6.**   Chimäre Alpha-Amylase nach Anspruch 1, in der Q einen N-terminalen Teil der allgemeinen Formel Ib umfaßt

(Ib)

$X_1$-Asn-Gly-Thr-Leu-Met-Gln-Tyr-Phe-Glu-Trp-Tyr-$X_2$-Pro-Asn-

Asp-Gly-Gln-His-Trp-Lys-Arg-Leu-Gln-Asn-Asp-$X_3$-Leu-$X_4$-Gly-

Ile-Thr-Ala-Val-Trp-Ile-Pro-Pro-Ala-Tyr-Lys-Gly-$X_5$-Ser-Gln-

$X_6$-Asp-$X_7$-Gly-Tyr-Gly;

in der

    $X_1$      Ala-Asn-Leu oder Val umfaßt,

X₂      Met oder Thr umfaßt,

X₃      Ser-Ala-Tyr oder Ala-Glu-His umfaßt,

X₄      Ala-Gly-His oder Ser-Asp-Ile umfaßt,

X₅      Thr oder Leu umfaßt,

X₆      Ala oder Ser umfaßt und

X₇      Val oder Asn umfaßt.

7.    Chimäre Alpha-Amylase nach Anspruch 6, in der

X₁      Val umfaßt,

X₂      Thr umfaßt,

X₃      Ala-Glu-His umfaßt,

X₄      Ser-Asp-Ile umfaßt,

X₅      Leu umfaßt,

X₆      Ser umfaßt und

X₇      Asn umfaßt.

8.    Alpha-Amylase nach Anspruch 4, 5, 6 oder 7, in der L einen C-terminalen Teil der allgemeinen Formel Ic umfaßt

$$(Ic)$$

```
              90                    95                   100
     Ser-Leu-His-Ser-Arg-Asp-Ile-Asn-Val-Tyr-Gly-Asp-Val

             105                   110                   115
 Val-Ile-Asn-His-Lys-Gly-Gly-Ala-Asp-Ala-Thr-Glu-Asp-Val-Thr-

             120                   125                   130
 Ala-Val-Glu-Val-Asp-Pro-Ala-Asp-Arg-Asn-Arg-Val-Ile-Ser-Gly-

             135                   140                   145
 Glu-His-Arg-Ile-Lys-Ala-Trp-Thr-His-Phe-His-Phe-Pro-Gly-Arg-

             150                   155                   160
 Gly-Ser-Thr-Tyr-Ser-Asp-Phe-Lys-Trp-His-Trp-Tyr-His-Phe-Asp-

             165                   170                   175
 Gly-Thr-Asp-Trp-Asp-Glu-Ser-Arg-Lys-Leu-Asn-Arg-Ile-Tyr-Lys-
```

```
                 180                    185                    190
     Phe-Gln-Gly-Lys-Ala-Trp-Asp-Trp-Glu-Val-Ser-Asn-Glu-Asn-Gly-

                 195                    200                    205
     Asn-Tyr-Asp-Tyr-Leu-Met-Tyr-Ala-Asp-Ile-Asp-Tyr-Asp-His-Pro-

                 210                    215                    220
     Asp-Val-Ala-Ala-Glu-Ile-Lys-Arg-Trp-Gly-Thr-Trp-Tyr-Ala-Asn-

                 225                    230                    235
     Glu-Leu-Gln-Leu-Asp-Gly-Phe-Arg-Leu-Asp-Ala-Val-Lys-His-Ile-

                 240                    245                    250
     Lys-Phe-Ser-Phe-Leu-Arg-Asp-Trp-Val-Asn-His-Val-Arg-Glu-Lys-

                 255                    260                    265
     Thr-Gly-Lys-Glu-Met-Phe-Thr-Val-Ala-Glu-Tyr-Trp-Gln-Asn-Asp-

                 270                    275                    280
     Leu-Gly-Ala-Leu-Glu-Asn-Tyr-Leu-Asn-Lys-Thr-Asn-Phe-Asn-His-

                  285                   290                    285
     Ser-Val-Phe-Asp-Val-Pro-Leu-His-Tyr-Gln-Phe-His-Ala-Ala-Ser-

                 300                    305                    310
     Thr-Gln-Gly-Gly-Gly-Tyr-Asp-Met-Arg-Lys-Leu-Leu-Asn-Ser-Thr-

                 315                    320                    325
     Val-Val-Ser-Lys-His-Pro-Leu-Lys-Ala-Val-Thr-Phe-Val-Asp-Asn-

                 330                    335                    340
     His-Asp-Thr-Gln-Pro-Gly-Gln-Ser-Leu-Glu-Ser-Thr-Val-Gln-Thr-

                 345                    350                    355
     Trp-Phe-Lys-Pro-Leu-Ala-Tyr-Ala-Phe-Ile-Leu-Thr-Arg-Glu-Ser-

                 360                    365                    370
     Gly-Tyr-Pro-Gln-Val-Phe-Tyr-Gly-Asp-Met-Tyr-Gly-Thr-Lys-Gly-

                 375                    380                    385
     Asp-Ser-Gln-Arg-Glu-Ile-Pro-Ala-Leu-Lys-His-Lys-Ile-Glu-Pro-

                 390                    395                    400
     Ile-Leu-Lys-Ala-Arg-Lys-Gln-Tyr-Ala-Tyr-Gly-Ala-Gln-His-Asp-

                 405                    410                    415
     Tyr-Phe-Asp-His-His-Asp-Ile-Val-Gly-Trp-Thr-Arg-Glu-Gly-Asp-

                 420                    425                    430
     Ser-Ser-Val-Ala-Asn-Ser-Gly-Leu-Ala-Ala-Leu-Ile-Thr-Asp-Gly-

                 435                    440                    445
     Pro-Gly-Gly-Ala-Lys-Arg-Met-Tyr-Val-Gly-Arg-Gln-Asn-Ala-Gly-
```

```
                450                          .455                         460
   Glu-Thr-Trp-His-Asp-Ile-Thr-Gly-Asn-Arg-Ser-Glu-Pro-Val-Val-

                465                          470                          475
   Ile-Asn-Ser-Glu-Gly-Trp-Gly-Glu-Phe-His-Val-Asn-Gly-Gly-Ser-

                480         483
   Val-Ser-Ile-Tyr-Val-Gln-Arg.
```

9.  Verfahren zur Herstellung einer chimären Alpha-Amylase, welches umfaßt:
    (a) daß die N-terminale Kodierungsregion des Alpha-Amylase-Gens von B. amyloliquefaciens mit der C-terminalen Kodierungsregion des Alpha-Amylase-Gens von B. licheniformis in vivo rekombiniert wird, um Rekombinanten zu bilden;
    (b) daß die Rekombinanten, die eine chimäre Alpha-Amylase produzieren, die hitzestabil ist und eine verringerte negative Wirkung auf die Verwendung von Glucoamylase aus A. niger und Pullulanase aus B. acidopullulyticus zur Verzuckerung von Stärke zeigt, selektiert werden;
    (c) daß die selektierten Rekombinanten in einem geeigneten Wachstumsmedium kultiviert werden und
    (d) daß die chimäre Alpha-Amylase aus der Kultur gewonnen wird.

10. Verfahren zur Umwandlung von Stärke in High-Dextrose-Sirup, welches umfaßt:
    (a) daß die Stärke mit der chimären Alpha-Amylase von Anspruch 1, 2, 3, 4, 5, 6 oder 7 zur Reaktion gebracht wird, um Oligosaccharide zu bilden; und
    (b) daß die in Schritt (a) gebildeten Oligosaccharide mit einer Glucoamylase zur Reaktion gebracht werden, um Dextrose zu bilden.

11. Verfahren zur Umwandlung von Stärke in High-Dextrose-Sirup, welches umfaßt:
    (a) daß die Stärke mit der chimären Alpha-Amylase von Anspruch 8 zur Reaktion gebracht wird, um Oligosaccharide zu bilden; und
    (b) daß die in Schritt (a) gebildeten Oligosaccharide mit einer Glucoamylase zur Reaktion gebracht werden, um Dextrose zu bilden.

**Patentansprüche für folgende Vertragsstaaten : AT, ES, GR**

1.  Verfahren zur Herstellung einer chimären Alpha-Amylase, welches umfaßt:
    a) daß die N-terminale Kodierungsregion des Alpha-Amylase-Gens von B. amyloliquefaciens mit der C-terminalen Kodierungsregion des Alpha-Amylase-Gens von B. licheniformis in vivo rekombiniert wird, um Rekombinanten zu bilden;
    b) daß die Rekombinanten, die eine chimäre Alpha-Amylase produzieren, die hitzestabil ist und eine verringerte negative Wirkung auf die Verwendung von Glucoamylase aus A. niger und Pullulanase aus B. acidopullulyticus zur Verzuckerung von Stärke zeigt, selektiert werden;
    c) daß die selektierten Rekombinanten in einem geeigneten Wachstumsmedium kultiviert werden und
    d) daß die chimäre Alpha-Amylase aus der Kultur gewonnen wird.

2.  Verfahren zur Umwandlung von Stärke in High-Dextrose-Sirup, welches umfaßt:
    a) daß die Stärke mit einer chimären Alpha-Amylase zur Reaktion gebracht wird, die hitzestabil ist und eine verringerte negative Wirkung auf die Verwendung von Glucoamylase aus A. niger und Pullulanase aus B. acidopullulyticus zur Verzuckerung von Stärke zeigt, mit der allgemeinen Formel I

```
                        (I)
                      Q-R-L
```

in der Q einen N-terminalen Teil mit von 55 bis 60 Aminosäureresten umfaßt, der wenigstens 75% homolog zu den 55 N-terminalen Aminosäureresten in der Alpha-Amylase aus Bacillus amyloliquefaciens ist, wie in Takkinen et al., J. Biol. Chem. 258 (1983), 1007-1013 beschrieben;
R einen Teil der allgemeinen Formel Ia umfaßt

(Ia)

58 60 70
Pro-Tyr-Asp-Leu-Tyr-Asp-Leu-Gly-Glu-Phe-$X_8$-Gln-Lys-

80
Gly-Thr-Val-Arg-Thr-Lys-Tyr-Gly-Thr-Lys-$X_9$-Glu-Leu

88
Gln-$X_{10}$-Ala-Ile-Lys

in der

$X_8$ His oder Gln umfaßt,

$X_9$ Gly oder Ser umfaßt,

$X_{10}$ Ser oder Asp umfaßt; und

L einen C-terminalen Teil mit von 390 bis 400 Aminosäureresten umfaßt, der wenigstens 75% homolog zu den 395 C-terminalen Aminosäureresten in der Alpha-Amylase aus Bacillus licheniformis 584 (ATCC 27811) ist, um Oligosaccharide zu bilden; und

(b) daß die in Schritt (a) gebildeten Oligosaccharide mit einer Glucoamylase zur Reaktion gebracht werden, um Dextrose zu bilden.

3. Verfahren nach Anspruch 2, dadurch gekennzeichnet, daß in der chimären Alpha-Amylase

$X_8$ His umfaßt,

$X_9$ Gly umfaßt und

$X_{10}$ Ser umfaßt.

4. Verfahren nach Anspruch 2, dadurch gekennzeichnet, daß in der chimären Alpha-Amylase

$X_8$ Gln umfaßt,

$X_9$ Ser umfaßt und

$X_{10}$ Asp umfaßt.

5. Verfahren nach Anspruch 2, dadurch gekennzeichnet, daß in der chimären Alpha-Amylase die Homologien wenigstens 80% sind.

6. Verfahren nach Anspruch 2, dadurch gekennzeichnet, daß in der chimären Alpha-Amylase die Homologien wenigstens 90% sind.

7. Verfahren nach Anspruch 2, dadurch gekennzeichnet, daß in der chimären Alpha-Amylase Q einen N-terminalen Teil der allgemeinen Formel Ib umfaßt

(Ib)

$$\begin{array}{ccc} & 5 & 10 & 15 \\ X_1\text{-Asn-Gly-Thr-Leu-Met-Gln-Tyr-Phe-Glu-Trp-Tyr-}X_2\text{-Pro-Asn-} \\ & 20 & 25 & 30 \quad 35 \\ \text{Asp-Gly-Gln-His-Trp-Lys-Arg-Leu-Gln-Asn-Asp-}X_3\text{-Leu-}X_4\text{-Gly-} \\ & 40 & 45 & 50 \\ \text{Ile-Thr-Ala-Val-Trp-Ile-Pro-Pro-Ala-Tyr-Lys-Gly-}X_5\text{-Ser-Gln-} \\ & 55 \\ X_6\text{-Asp-}X_7\text{-Gly-Tyr-Gly;} \end{array}$$

in der

$X_1$  Ala-Asn-Leu oder Val umfaßt,

$X_2$  Met oder Thr umfaßt,

$X_3$  Ser-Ala-Tyr oder Ala-Glu-His umfaßt,

$X_4$  Ala-Gly-His oder Ser-Asp-Ile umfaßt,

$X_5$  Thr oder Leu umfaßt,

$X_5$  Ala oder Ser umfaßt und

$X_7$  Val oder Asn umfaßt.

8.  Verfahren nach Anspruch 7, dadurch gekennzeichnet, daß in der chimären Alpha-Amylase

$X_1$  Val umfaßt,

$X_2$  Thr umfaßt,

$X_3$  Ala-Glu-His umfaßt,

$X_4$  Ser-Asp-Ile umfaßt,

$X_5$  Leu umfaßt,

$X_5$  Ser umfaßt und

$X_7$  Asn umfaßt.

9.  Verfahren zur Umwandlung von Stärke in High-Dextrose-Sirup, welches umfaßt:

(a) daß die Stärke mit einer chimären Alpha-Amylase zur Reaktion gebracht wird, um Oligosaccharide zu bilden; und

(b) daß die in Schritt (a) gebildeten Oligosaccharide mit einer Glucoamylase zur Reaktion gebracht werden, um Dextrose zu bilden, wobei die chimäre Alpha-Amylase so ist, wie in Anspruch 5, 6, 7 oder 8 definiert, und in der L einen C-terminalen Teil der allgemeinen Formel Ic umfaßt

25

(Ic)

```
            90                  95                  100
    Ser-Leu-His-Ser-Arg-Asp-Ile-Asn-Val-Tyr-Gly-Asp-Val

            105                 110                 115
 Val-Ile-Asn-His-Lys-Gly-Gly-Ala-Asp-Ala-Thr-Glu-Asp-Val-Thr-

            120                 125                 130
 Ala-Val-Glu-Val-Asp-Pro-Ala-Asp-Arg-Asn-Arg-Val-Ile-Ser-Gly-

            135                 140                 145
 Glu-His-Arg-Ile-Lys-Ala-Trp-Thr-His-Phe-His-Phe-Pro-Gly-Arg-

            150                 155                 160
 Gly-Ser-Thr-Tyr-Ser-Asp-Phe-Lys-Trp-His-Trp-Tyr-His-Phe-Asp-

            165                 170                 175
 Gly-Thr-Asp-Trp-Asp-Glu-Ser-Arg-Lys-Leu-Asn-Arg-Ile-Tyr-Lys-
 ____

            180                 185                 190
 Phe-Gln-Gly-Lys-Ala-Trp-Asp-Trp-Glu-Val-Ser-Asn-Glu-Asn-Gly-

            195                 200                 205
 Asn-Tyr-Asp-Tyr-Leu-Met-Tyr-Ala-Asp-Ile-Asp-Tyr-Asp-His-Pro-

            210                 215                 220
 Asp-Val-Ala-Ala-Glu-Ile-Lys-Arg-Trp-Gly-Thr-Trp-Tyr-Ala-Asn-

            225                 230                 235
 Glu-Leu-Gln-Leu-Asp-Gly-Phe-Arg-Leu-Asp-Ala-Val-Lys-His-Ile-

            240                 245                 250
 Lys-Phe-Ser-Phe-Leu-Arg-Asp-Trp-Val-Asn-His-Val-Arg-Glu-Lys-

            255                 260                 265
 Thr-Gly-Lys-Glu-Met-Phe-Thr-Val-Ala-Glu-Tyr-Trp-Gln-Asn-Asp-
```

```
                270                    275                    280
  Leu-Gly-Ala-Leu-Glu-Asn-Tyr-Leu-Asn-Lys-Thr-Asn-Phe-Asn-His-

               285                    290                    285
  Ser-Val-Phe-Asp-Val-Pro-Leu-His-Tyr-Gln-Phe-His-Ala-Ala-Ser-

               300                    305                    310
  Thr-Gln-Gly-Gly-Gly-Tyr-Asp-Met-Arg-Lys-Leu-Leu-Asn-Ser-Thr-

                315                    320                    325
  Val-Val-Ser-Lys-His-Pro-Leu-Lys-Ala-Val-Thr-Phe-Val-Asp-Asn-

               330                    335                    340
  His-Asp-Thr-Gln-Pro-Gly-Gln-Ser-Leu-Glu-Ser-Thr-Val-Gln-Thr-

               345                    350                    355
  Trp-Phe-Lys-Pro-Leu-Ala-Tyr-Ala-Phe-Ile-Leu-Thr-Arg-Glu-Ser-

               360                    365                    370
  Gly-Tyr-Pro-Gln-Val-Phe-Tyr-Gly-Asp-Met-Tyr-Gly-Thr-Lys-Gly-

               375                    380                    385
  Asp-Ser-Gln-Arg-Glu-Ile-Pro-Ala-Leu-Lys-His-Lys-Ile-Glu-Pro-

               390                    395                    400
  Ile-Leu-Lys-Ala-Arg-Lys-Gln-Tyr-Ala-Tyr-Gly-Ala-Gln-His-Asp-

               405                    410                    415
  Tyr-Phe-Asp-His-His-Asp-Ile-Val-Gly-Trp-Thr-Arg-Glu-Gly-Asp-

               420                    425                    430
  Ser-Ser-Val-Ala-Asn-Ser-Gly-Leu-Ala-Ala-Leu-Ile-Thr-Asp-Gly-

               435                    440                    445
  Pro-Gly-Gly-Ala-Lys-Arg-Met-Tyr-Val-Gly-Arg-Gln-Asn-Ala-Gly-

               450                    455                    460
  Glu-Thr-Trp-His-Asp-Ile-Thr-Gly-Asn-Arg-Ser-Glu-Pro-Val-Val-

               465                    470                    475
  Ile-Asn-Ser-Glu-Gly-Trp-Gly-Glu-Phe-His-Val-Asn-Gly-Gly-Ser-

               480         483
  Val-Ser-Ile-Tyr-Val-Gln-Arg.
```

## Revendications
**Revendications pour les Etats contractants suivants : BE, CH, DE, FR, GB, IT, LI, LU, NL, SE**

1. Alpha-amylase chimère, qui est thermostable et manifeste un effet négatif réduit sur l'utilisation de la glucoamylase de A. niger et de la pullulanase de B. acidopullulyticus pour la saccharification de l'amidon, ayant la formule générale I

$$(I)$$

$$Q-R-L$$

dans laquelle Q comprend une partie N-terminale de 55 à 60 résidus d'aminoacides qui a une homologie d'au moins 75 % avec les 55 résidus d'aminoacides N-terminaux de l'alpha-amylase de Bacillus amyloliquefaciens telle que décrite dans Takkinen et coll., J. Biol. Chem. 258 (1983) 1007-1013;

R comprend une partie de formule générale Ia

(Ia)

58          60                                                          70
Pro-Tyr-Asp-Leu-Tyr-Asp-Leu-Gly-Glu-Phe-$X_8$-Gln-Lys-
                                                    80
Gly-Thr-Val-Arg-Thr-Lys-Tyr-Gly-Thr-Lys-$X_9$-Glu-Leu
              88
Gln-$X_{10}$-Ala-Ile-Lys

dans laquelle

$X_8$ comprend His ou Gln,

$X_9$ comprend Gly ou Ser,

$X_{10}$ comprend Ser ou Asp; et

L comprend une partie C-terminale de 390 à 400 résidus d'aminoacides qui a une homologie d'au moins 75 % avec les 395 résidus d'aminoacides C-terminaux de l'alpha-amylase de Bacillus licheniformis 584 (ATCC 27811).

2. Alpha-amylase chimère selon la revendication 1, dans laquelle

$X_8$ comprend His,

$X_9$ comprend Gly, et

$X_{10}$ comprend Ser.

3. Alpha-amylase chimère selon la revendication 1, dans laquelle

$X_8$ comprend Gln,

$X_9$ comprend Ser, et

$X_{10}$ comprend Asp.

4. Alpha-amylase chimère selon la revendication 1, dans laquelle les homologies sont d'au moins 80 %.

5. Alpha-amylase chimère selon la revendication 1, dans laquelle les homologies sont d'au moins 90 %.

6. Alpha-amylase chimère selon la revendication 1, dans laquelle Q comprend une partie N-terminale de formule générale Ib

(Ib)

          5                        10                        15
$X_1$-Asn-Gly-Thr-Leu-Met-Gln-Tyr-Phe-Glu-Trp-Tyr-$X_2$-Pro-Asn-
          20                        25                  30        35
Asp-Gly-Gln-His-Trp-Lys-Arg-Leu-Gln-Asn-Asp-$X_3$-Leu-$X_4$-Gly-
                    40                        45                        50
Ile-Thr-Ala-Val-Trp-Ile-Pro-Pro-Ala-Tyr-Lys-Gly-$X_5$-Ser-Gln-
                    55
$X_6$-Asp-$X_7$-Gly-Tyr-Gly;

28

dans laquelle
$X_1$ comprend Ala-Asn-Leu ou Val,
$X_2$ comprend Met ou Thr,
$X_3$ comprend Ser-Ala-Tyr ou Ala-Glu-His,
$X_4$ comprend Ala-Gly-His ou Ser-Asp-Ile,
$X_5$ comprend Thr ou Leu,
$X_5$ comprend Ala ou Ser, et
$X_7$ comprend Val ou Asn.

7. Alpha-amylase chimère selon la revendication 6, dans laquelle
$X_1$ comprend Val,
$X_2$ comprend Thr,
$X_3$ comprend Ala-Glu-His,
$X_4$ comprend Ser-Asp-Ile,
$X_5$ comprend Leu,
$X_5$ comprend Ser, et
$X_7$ comprend Asn.

8. Alpha-amylase selon la revendication 4, 5, 6 ou 7, dans laquelle L comprend une partie C-terminale de formule générale Ic

29

(Ic)

```
          90                    95                   100
     Ser-Leu-His-Ser-Arg-Asp-Ile-Asn-Val-Tyr-Gly-Asp-Val

          105                   110                  115
Val-Ile-Asn-His-Lys-Gly-Gly-Ala-Asp-Ala-Thr-Glu-Asp-Val-Thr-

          120                   125                  130
Ala-Val-Glu-Val-Asp-Pro-Ala-Asp-Arg-Asn-Arg-Val-Ile-Ser-Gly-

          135                   140                  145
Glu-His-Arg-Ile-Lys-Ala-Trp-Thr-His-Phe-His-Phe-Pro-Gly-Arg-

          150                   155                  160
Gly-Ser-Thr-Tyr-Ser-Asp-Phe-Lys-Trp-His-Trp-Tyr-His-Phe-Asp-

          165                   170                  175
Gly-Thr-Asp-Trp-Asp-Glu-Ser-Arg-Lys-Leu-Asn-Arg-Ile-Tyr-Lys-

          180                   185                  190
Phe-Gln-Gly-Lys-Ala-Trp-Asp-Trp-Glu-Val-Ser-Asn-Glu-Asn-Gly-

          195                   200                  205
Asn-Tyr-Asp-Tyr-Leu-Met-Tyr-Ala-Asp-Ile-Asp-Tyr-Asp-His-Pro-

          210                   215                  220
Asp-Val-Ala-Ala-Glu-Ile-Lys-Arg-Trp-Gly-Thr-Trp-Tyr-Ala-Asn-

          225                   230                  235
Glu-Leu-Gln-Leu-Asp-Gly-Phe-Arg-Leu-Asp-Ala-Val-Lys-His-Ile-

          240                   245                  250
Lys-Phe-Ser-Phe-Leu-Arg-Asp-Trp-Val-Asn-His-Val-Arg-Glu-Lys-

          255                   260                  265
Thr-Gly-Lys-Glu-Met-Phe-Thr-Val-Ala-Glu-Tyr-Trp-Gln-Asn-Asp-

          270                   275                  280
Leu-Gly-Ala-Leu-Glu-Asn-Tyr-Leu-Asn-Lys-Thr-Asn-Phe-Asn-His-

          285                   290                  285
Ser-Val-Phe-Asp-Val-Pro-Leu-His-Tyr-Gln-Phe-His-Ala-Ala-Ser-

          300                   305                  310
Thr-Gln-Gly-Gly-Gly-Tyr-Asp-Met-Arg-Lys-Leu-Leu-Asn-Ser-Thr-
```

```
            315                  320                  325
Val-Val-Ser-Lys-His-Pro-Leu-Lys-Ala-Val-Thr-Phe-Val-Asp-Asn-

            330                  335                  340
His-Asp-Thr-Gln-Pro-Gly-Gln-Ser-Leu-Glu-Ser-Thr-Val-Gln-Thr-

            345                  350                  355
Trp-Phe-Lys-Pro-Leu-Ala-Tyr-Ala-Phe-Ile-Leu-Thr-Arg-Glu-Ser-

            360                  365                  370
Gly-Tyr-Pro-Gln-Val-Phe-Tyr-Gly-Asp-Met-Tyr-Gly-Thr-Lys-Gly-

            375                  380                  385
Asp-Ser-Gln-Arg-Glu-Ile-Pro-Ala-Leu-Lys-His-Lys-Ile-Glu-Pro-

            390                  395                  400
Ile-Leu-Lys-Ala-Arg-Lys-Gln-Tyr-Ala-Tyr-Gly-Ala-Gln-His-Asp-

            405                  410                  415
Tyr-Phe-Asp-His-His-Asp-Ile-Val-Gly-Trp-Thr-Arg-Glu-Gly-Asp-

            420                  425                  430
Ser-Ser-Val-Ala-Asn-Ser-Gly-Leu-Ala-Ala-Leu-Ile-Thr-Asp-Gly-

            435                  440                  445
Pro-Gly-Gly-Ala-Lys-Arg-Met-Tyr-Val-Gly-Arg-Gln-Asn-Ala-Gly-

            450                  455                  460
Glu-Thr-Trp-His-Asp-Ile-Thr-Gly-Asn-Arg-Ser-Glu-Pro-Val-Val-

            465                  470                  475
Ile-Asn-Ser-Glu-Gly-Trp-Gly-Glu-Phe-His-Val-Asn-Gly-Gly-Ser-

            480         483
Val-Ser-Ile-Tyr-Val-Gln-Arg.
```

9. Procédé de production d'une alpha-amylase chimère dans lequel:

(a) on effectue la recombinaison in vivo de la région codante N-terminale du gène de l'alpha-amylase de B. amyloliquefaciens avec la région codante C-terminale du gène de l'alpha-amylase de B. licheniformis pour former des recombinés;

(b) on sélectionne les recombinés qui produisent une alpha-amylase chimère qui est thermostable et manifeste un effet négatif réduit sur l'utilisation de la glucoamylase d'A. niger et de la pullulanase de B. acidopullulyticus pour la saccharification de l'amidon;

(c) on cultive les recombinés sélectionnés dans un milieu de croissance approprié, et

(d) on récupère l'alpha-amylase chimère à partir de la culture.

10. Procédé de conversion d'amidon en sirop à haute teneur en dextrose, dans lequel:

(a) on fait réagir de l'amidon avec l'alpha-amylase chimère de la revendication 1, 2, 3, 4, 5, 6 ou 7 pour former des oligosaccharides; et

(b) on fait réagir les oligosaccharides formés dans l'étape (a) avec une glucoamylase pour former du dextrose.

11. Procédé de conversion d'amidon en sirop à haute teneur en dextrose, selon lequel:

(a) on fait réagir de l'amidon avec l'alpha-amylase chimère de la revendication 8 pour former des oligosaccharides; et

(b) on fait réagir les oligosaccharides formés dans l'étape (a) avec une glucoamylase pour former du dextrose.

**Revendications pour les Etats contractants suivants : AT, ES, GR**

1. Procédé pour la production d'une alpha-amylase chimère comprenant :

    (a) la recombinaison in vivo de la région codante N-terminale du gène de l'alpha-amylase de B. amyloliquefaciens avec la région codante C-terminale du gène de l'alpha-amylase de B. licheniformis pour former des recombinés ;

    (b) la sélection des recombinés qui produisent une alpha-amylase chimère qui est thermostable et manifeste un effet négatif réduit sur l'utilisation de la glucoamylase d'A. niger et de la pullulanase de B. acidopullulyticus pour la saccharification de l'amidon ;

    (c) la mise en culture des recombinés sélectionnés dans un milieu de croissance approprié, et

    (d) la récupération de l'alpha-amylase chimère de la culture.

2. Procédé pour la conversion d'amidon en sirop à forte teneur en dextrose, comprenant :

    (a) la mise en réaction de l'amidon avec une alpha-amylase chimère qui est thermostable et manifeste un effet négatif réduit sur l'utilisation de la glucoamylase d'A. niger et de la pullulanase de B. acidopullulyticus ayant la formule générale I

$$(I)$$

$$Q-R-L$$

dans laquelle

    Q comprend une partie N-terminale de 55 à 60 résidus d'aminoacides qui a une homologie d'au moins 75 % avec les 55 résidus d'aminoacides N-terminaux de l'alpha-amylase de Bacillus amyloliquefaciens telle que décrite dans Takkinen et al., J. Biol. Chem. 258 (1983) 1007-1013 ;

    R comprend une partie de formule générale Ia

$$(Ia)$$

$$\overset{58}{\text{Pro}}-\text{Tyr}-\text{Asp}-\text{Leu}-\overset{60}{\text{Tyr}}-\text{Asp}-\text{Leu}-\text{Gly}-\text{Glu}-\text{Phe}-X_8-\text{Gln}-\overset{70}{\text{Lys}}-$$

$$\text{Gly}-\text{Thr}-\text{Val}-\text{Arg}-\text{Thr}-\text{Lys}-\text{Tyr}-\text{Gly}-\text{Thr}-\overset{80}{\text{Lys}}-X_9-\text{Glu}-\text{Leu}$$

$$\overset{88}{\text{Gln}}-X_{10}-\text{Ala}-\text{Ile}-\text{Lys}$$

dans laquelle

    $X_8$      comprend His ou Gln,

    $X_9$      comprend Gly ou Ser,

    $X_{10}$     comprend Ser ou Asp ; et

    L comprend une partie C-terminale de 390 à 400 résidus d'aminoacides qui a une homologie d'au moins 75 % avec les 395 résidus d'aminoacides C-terminaux de l'alpha-amylase de Bacillus licheniformis 584 (ATCC 27811), pour former des oligosaccharides ; et

    (b) on met en réaction les oligosaccharides formés dans l'étape (a) avec une glucoamylase pour former du dextrose.

3. Procédé selon la revendication 2, dans lequel dans l'alpha-amylase chimère,

    $X_8$      comprend His,

    $X_9$      comprend Gly, et

    $X_{10}$     comprend Ser.

4. Procédé selon la revendication 2, dans lequel dans l'alpha-amylase chimère,
   $X_8$       comprend Gln,
   $X_9$       comprend Ser, et
   $X_{10}$      comprend Asp.

5. Procédé selon la revendication 2, dans lequel dans l'alpha-amylase chimère, les homologies sont d'au moins 80 %.

6. Procédé selon la revendication 2, dans lequel dans l'alpha-amylase chimère, les homologies sont d'au moins 90 %.

7. Procédé selon la revendication 2, dans lequel dans l'alpha-amylase chimère, Q comprend une partie N-terminale de formule générale Ib

$$(Ib)$$

$$
\begin{array}{l}
\quad\quad\quad 5 \quad\quad\quad\quad\quad\quad 10 \quad\quad\quad\quad\quad\quad\quad 15 \\
X_1\text{-Asn-Gly-Thr-Leu-Met-Gln-Tyr-Phe-Glu-Trp-Tyr-}X_2\text{-Pro-Asn-} \\
\quad\quad\quad 20 \quad\quad\quad\quad\quad\quad 25 \quad\quad\quad\quad 30 \quad\quad 35 \\
\text{Asp-Gly-Gln-His-Trp-Lys-Arg-Leu-Gln-Asn-Asp-}X_3\text{-Leu-}X_4\text{-Gly-} \\
\quad\quad\quad\quad 40 \quad\quad\quad\quad\quad\quad 45 \quad\quad\quad\quad\quad\quad 50 \\
\text{Ile-Thr-Ala-Val-Trp-Ile-Pro-Pro-Ala-Tyr-Lys-Gly-}X_5\text{-Ser-Gln-} \\
\quad\quad\quad\quad 55 \\
X_6\text{-Asp-}X_7\text{-Gly-Tyr-Gly;}
\end{array}
$$

dans laquelle
$X_1$       comprend Ala-Asn-Leu ou Val,
$X_2$       comprend Met ou Thr,
$X_3$       comprend Ser-Ala-Tyr ou Ala-Glu-His,
$X_4$       comprend Ala-Gly-His ou Ser-Asp-Ile,
$X_8$       comprend Thr ou Leu,
$X_8$       comprend Ala ou Ser, et
$X_7$       comprend Val ou Asn.

8. Procédé selon la revendication 7, dans lequel, dans l'alpha-amylase chimère,
   $X_1$       comprend Val,
   $X_2$       comprend Thr,
   $X_3$       comprend Ala-Glu-His,
   $X_4$       comprend Ser-Asp-Ile,
   $X_8$       comprend Leu,
   $X_8$       comprend Ser, et
   $X_7$       comprend Asn.

9. Procédé pour la conversion d'amidon en sirop à forte teneur en dextrose, comprenant :
   (a) la mise en réaction de l'amidon avec une alpha-amylase chimère pour former des oligosaccharides ; et
   (b) la mise en réaction des oligosaccharides formés à l'étape (a) avec une glucoamylase pour former du dextrose, l'alpha-amylase chimère étant telle que définie dans les revendications 5, 6, 7 ou 8 et dans laquelle L comprend une partie C-terminale de formule générale Ic

(Ic)

```
              90                    95                   100
        Ser-Leu-His-Ser-Arg-Asp-Ile-Asn-Val-Tyr-Gly-Asp-Val

              105                   110                  115
   Val-Ile-Asn-His-Lys-Gly-Gly-Ala-Asp-Ala-Thr-Glu-Asp-Val-Thr-

              120                   125                  130
   Ala-Val-Glu-Val-Asp-Pro-Ala-Asp-Arg-Asn-Arg-Val-Ile-Ser-Gly-

              135                   140                  145
   Glu-His-Arg-Ile-Lys-Ala-Trp-Thr-His-Phe-His-Phe-Pro-Gly-Arg-

              150                   155                  160
   Gly-Ser-Thr-Tyr-Ser-Asp-Phe-Lys-Trp-His-Trp-Tyr-His-Phe-Asp-

              165                   170                  175
   Gly-Thr-Asp-Trp-Asp-Glu-Ser-Arg-Lys-Leu-Asn-Arg-Ile-Tyr-Lys-

              180                   185                  190
   Phe-Gln-Gly-Lys-Ala-Trp-Asp-Trp-Glu-Val-Ser-Asn-Glu-Asn-Gly-

              195                   200                  205
   Asn-Tyr-Asp-Tyr-Leu-Met-Tyr-Ala-Asp-Ile-Asp-Tyr-Asp-His-Pro-

              210                   215                  220
   Asp-Val-Ala-Ala-Glu-Ile-Lys-Arg-Trp-Gly-Thr-Trp-Tyr-Ala-Asn-

              225                   230                  235
   Glu-Leu-Gln-Leu-Asp-Gly-Phe-Arg-Leu-Asp-Ala-Val-Lys-His-Ile-

              240                   245                  250
   Lys-Phe-Ser-Phe-Leu-Arg-Asp-Trp-Val-Asn-His-Val-Arg-Glu-Lys-

              255                   260                  265
   Thr-Gly-Lys-Glu-Met-Phe-Thr-Val-Ala-Glu-Tyr-Trp-Gln-Asn-Asp-

              270                   275                  280
   Leu-Gly-Ala-Leu-Glu-Asn-Tyr-Leu-Asn-Lys-Thr-Asn-Phe-Asn-His-

              285                   290                  285
   Ser-Val-Phe-Asp-Val-Pro-Leu-His-Tyr-Gln-Phe-His-Ala-Ala-Ser-
```

```
                    300                     305                     310
Thr-Gln-Gly-Gly-Gly-Tyr-Asp-Met-Arg-Lys-Leu-Leu-Asn-Ser-Thr

                    315                     320                     325
Val-Val-Ser-Lys-His-Pro-Leu-Lys-Ala-Val-Thr-Phe-Val-Asp-Asn-

                    330                     335                     340
His-Asp-Thr-Gln-Pro-Gly-Gln-Ser-Leu-Glu-Ser-Thr-Val-Gln-Thr-

                    345                     350                     355
Trp-Phe-Lys-Pro-Leu-Ala-Tyr-Ala-Phe-Ile-Leu-Thr-Arg-Glu-Ser-

                    360                     365                     370
Gly-Tyr-Pro-Gln-Val-Phe-Tyr-Gly-Asp-Met-Tyr-Gly-Thr-Lys-Gly-

                    375                     380                     385
Asp-Ser-Gln-Arg-Glu-Ile-Pro-Ala-Leu-Lys-His-Lys-Ile-Glu-Pro-

                    390                     395                     400
Ile-Leu-Lys-Ala-Arg-Lys-Gln-Tyr-Ala-Tyr-Gly-Ala-Gln-His-Asp-

                    405                     410                     415
Tyr-Phe-Asp-His-His-Asp-Ile-Val-Gly-Trp-Thr-Arg-Glu-Gly-Asp-

                    420                     425                     430
Ser-Ser-Val-Ala-Asn-Ser-Gly-Leu-Ala-Ala-Leu-Ile-Thr-Asp-Gly-

                    435                     440                     445
Pro-Gly-Gly-Ala-Lys-Arg-Met-Tyr-Val-Gly-Arg-Gln-Asn-Ala-Gly-

                    450                     455                     460
Glu-Thr-Trp-His-Asp-Ile-Thr-Gly-Asn-Arg-Ser-Glu-Pro-Val-Val-

                    465                     470                     475
Ile-Asn-Ser-Glu-Gly-Trp-Gly-Glu-Phe-His-Val-Asn-Gly-Gly-Ser-

                    480           483
Val-Ser-Ile-Tyr-Val-Gln-Arg.
```

B. LICHENIFORMIS

B. AMYLOLIQUEFACIENS

QL1864

FIG. 1

EP 0 252 666 B1

FIG. 2

pDN 1822   Amy⁺; 8.0 kb

pDN 1850
Amy⁻; 6.6 kb

FIG. 3

FIG. 4

pDN 1864   Amy⁺;   5.4 kb

37